# EUROPÄISCHE PATENTSCHRIFT

(11) **EP 2 505 156 B1**
(45) Veröffentlichungstag und Bekanntmachung des Hinweises auf die Patenterteilung: **23.07.2014**
(21) Anmeldenummer: 12161168.5
(22) Anmeldetag: 26.03.2012
(51) Int. Cl.: A61B 17/70

(54) **Facettengelenkimplantat**
Facet joint implant
Implant pour articulation à facettes

(30) Priorität: 28.03.2011 DE 102011001590
(43) Veröffentlichungstag der Anmeldung: 03.10.2012
(73) Patentinhaber: Aesculap AG, 78532 Tuttlingen (DE)
(72) Erfinder: Schulze, Martin, 48149 Münster (DE); Kettenberger, Ulrike, 83308 Trostberg (DE)
(74) Vertreter: Hoeger, Stellrecht & Partner Patentanwälte

(56) Entgegenhaltungen:
- WO-A2-2006/009855
- DE-A1-102007 051 783
- US-A1- 2006 190 081
- US-A1- 2010 131 008

## Beschreibung

Die vorliegende Erfindung betrifft ein Facettengelenkimplantat umfassend ein Gelenkflächenersatzimplantat und eine Befestigungseinrichtung zum Festlegen des Gelenkflächenersatzimplantats im Gelenkspalt eines Facettengelenks einer menschlichen oder tierischen Wirbelsäule, welche Befestigungseinrichtung mindestens zwei vom Gelenkflächenersatzimplantat abstehende Befestigungselemente umfasst.

Facettengelenke können bei der Entstehung von Rückenschmerzen eine bedeutende Ursache darstellen. Der Ersatz von Facettengelenken aufgrund Degeneration oder Schädigung ist daher ein wichtiges Thema in der Orthopädie, insbesondere in der Wirbelsäulenchirurgie. In vielen Fällen ist eine Facettengelenkarthrose oder -degeneration eine natürliche Folge eines Höhenverlustes einer dem betroffenen Facettengelenk zugeordneten, das heißt insbesondere benachbarten, degenerierten Bandscheibe mit einer veränderten Lastverteilung auf ein zwischen zwei benachbarten Wirbeln der Wirbelsäule ausgebildeten Bewegungssegment. Das veränderte Facettengelenk kann jedoch auch eine iatrogene Erkrankung in Folge einer spinalen Dekompression oder des Einsatzes einer Bandscheibenprothese darstellen.

Zur Behandlung einer Facettengelenkarthrose oder -degeneration kann ein Ersatz des gesamten Facettengelenks oder zumindest der Gelenkflächen nach Entfernung der Schmerzen verursachenden Gelenkkapseln und Knorpelschicht vorgesehen werden. Da die Facettengelenke in den Bewegungssegmenten der Wirbelsäule eine wichtige Funktion bei der Lastübertragung und der Begrenzung des Bewegungsumfanges besonders in axialer Rotation und Flexion übernehmen, erscheint eine Erhaltung des natürlichen Bewegungsumfanges ("Rage of motion") und der natürlichen Lastverteilung innerhalb des Bewegungssegmentes für den Erfolg eines derartigen Implantates wichtig zu sein.

Allerdings stellt die Fixierung bei allen derartigen Implantaten eine besondere Herausforderung dar, da insbesondere die Primärstabilität wesentlich die Funktionsfähigkeit des Implantats nach dessen Implantation bedingt.

Facettengelenkimplantate der eingangs beschriebenen Art sind beispielsweise aus der WO 2009/006455 A1 sowie der US 2005/0055096 A1 bekannt. Diese Facettengelenkimplantate werden insbesondere mit Schrauben oder anderen Befestigungselementen am Knochen fixiert. Eine solche Befestigung des Facettengelenkimplantats ist jedoch zeitaufwendig und birgt Risiken. Insbesondere geht mit den genannten Befestigungselementen stets eine Schädigung benachbarter Knochensubstanz einher.

Es ist daher eine Aufgabe der vorliegenden Erfindung, ein Facettengelenkimplantat der eingangs beschriebenen Art so zu verbessern, dass es einfach und sicher an der Wirbelsäule festlegbar ist.

Diese Aufgabe wird bei einem Facettengelenkimplantat der eingangs beschriebenen Art erfindungsgemäß dadurch gelöst, dass die mindestens zwei Befestigungselemente im Bereich ihrer freien Enden jeweils mit mindestens einer Fixierelementdurchbrechung versehen sind und dass das Facettengelenkimplantat ein Fixierelement umfasst, welches durch mindestens zwei Fixierelementdurchbrechungen von mindestens zwei Befestigungselementen durchgeführt ist und dessen freie Enden miteinander verbindbar sind.

Ein derartiges, erfindungsgemäßes Facettengelenkimplantat ermöglicht eine optimale Anpassung an die knöchernen Strukturen im Bereich des zu behandelnden Facettengelenks. Die Befestigung des Facettengelenkimplantats ist einfach, da es insbesondere möglich ist, es nur aus zwei Teilen auszubilden. Beispielsweise kann das Fixierelement durch die Fixierelementdurchbrechungen aller Befestigungselemente durchgeführt und dann auf einfache Weise mit sich selbst verbunden werden. Dies gestattet eine schnelle, einfache und sichere Anbringung des Facettengelenkimplantats am vorhandenen Knochen, ohne diesen zu schädigen. Zusätzliche Befestigungselemente, die in den Knochen eingetrieben werden, sind nicht zwingend erforderlich, können aber bei Bedarf auch vorgesehen werden. Ferner ist es mit dem Facettengelenkimplantat insbesondere auch möglich, häufig an Facettengelenke angrenzende neurale Strukturen nicht zu gefährden. Des Weiteren kann es die vorgeschlagene Befestigungseinrichtung insbesondere auch verhindern, dass das Facettengelenkimplantat von den knöchernen Strukturen abrutschen kann. Grundsätzlich ist es auch denkbar, das erfindungsgemäß ausgebildete Facettengelenkimplantat durch einen minimalinvasiven Zugang in den Körper des Patienten einzuführen. Durch die einfache Befestigung des Facettengelenkimplantats sind Kombinationen desselben mit anderen Stabilisierungsmaßnahmen an der Wirbelsäule, insbesondere im Bereich des betroffenen Bewegungssegments, praktisch ohne Probleme möglich. Da zudem nicht zwingend knöcherne Strukturen bei der Festlegung des erfindungsgemäßen Facettengelenkimplantats geschädigt oder zerstört werden müssen, ist eine spätere Fusion benachbarter Wirbel nach Einsatz des vorgeschlagenen Facettengelenkimplantats immer noch möglich. Und schließlich ermöglicht es das vorgeschlagene Facettengelenkimplantat, insbesondere auch physiologische Gegebenheiten eines Patienten zu berücksichtigen, um eine möglichst schonende Befestigung des Facettengelenkimplantats zu erreichen.

Günstig ist es, wenn das Fixierelement flexibel ausgebildet ist. So ist es insbesondere möglich, das Facettengelenkimplantat an nahezu jede erdenkliche Gelenkfläche anzupassen und an dieser festzulegen. Ferner wird auch die Handhabung des Facettengelenkimplantats sehr einfach.

Vorzugsweise ist das Fixierelement in Form eines Fadens oder eines Drahts ausgebildet. Ein solches Fixierelement gestattet eine einfache Handhabung und auch eine Befestigung des Facettengelenkimplantats beziehungsweise eine Verriegelung desselben an knöchernen Strukturen der Wirbelsäulen von dorsal.

Besonders vorteilhaft ist es, wenn die mindestens zwei Befestigungselemente in Form von Befestigungslaschen, -lappen, -streifen, -schlaufen oder -ösen ausgebildet sind. Die Herstellung derartiger Befestigungselemente ist besonders einfach. Ferner gestatten sie es auf einfache Weise, gegebenenfalls noch erforderliche Fixierelementdurchbrechungen vorzusehen und so auszubilden, dass das Fixierelement sicher durch diese hindurchgeführt werden kann.

Günstig ist es, wenn freie Enden der mindestens zwei Befestigungselemente verdickt und/oder verstärkt ausgebildet sind oder wenn die mindestens zwei Befestigungselemente im Bereich ihrer freien Enden verdickt und/oder verstärkt ausgebildet sind. Derart geformte Befestigungselemente können insbesondere eine für den Einsatz des Facettengelenkimplantats dauerhafte Stabilität sicherstellen. Ein Ausreißen der Befestigungselemente durch eine Zugbeanspruchung durch das Fixierelement kann so einfach und sicher verhindert werden.

Das Ausreißen des Facettengelenkimplantats im Bereich der Fixierelementdurchbrechungen kann in vorteilhafter Weise insbesondere dadurch verhindert werden, dass die Fixierelementdurchbrechungen verdickt und/oder verstärkt ausgebildet sind.

Gemäß einer weiteren bevorzugten Ausführungsform der Erfindung kann ein Fixierelementverschluss zum zugbelastbaren Verbinden freier Enden des Fixierelements vorgesehen sein. Mit dem Fixierelementverschluss ist es auf einfache Weise möglich, freie Enden des Fixierelements zugbelastet miteinander zu verbinden, um das Facettengelenkimplantat an den vorhandenen knöchernen Strukturen der Wirbelsäule festzulegen.

Besonders einfach, schnell und sicher kann das Facettengelenkimplantat an der Wirbelsäule festgelegt werden, wenn der Fixierelementverschluss in Form eines Knotens, einer Plombe oder einer Klammer ausgebildet ist. Vorzugsweise umfasst das Facettengelenkimplantat vier bis 25 Befestigungselement. Je mehr Befestigungselemente vorgesehen sind, umso optimaler lässt sich das Facettengelenkimplantat an vorhandenen knöchernen Strukturen festlegen. Insbesondere ein enges Anliegen der Befestigungselemente am Knochen verhindert die Beeinträchtigung umliegender Strukturen, beispielsweise von Nervenwurzeln.

Des Weiteren kann es vorteilhaft sein, wenn die mindestens zwei Befestigungselemente unterschiedlich lang und/oder breit und/oder dick ausgebildet sind. So kann das Facettengelenkimplantat noch besser an Knochenstrukturen der Wirbelsäule angepasst werden. Günstig kann es insbesondere auch sein, wenn die mindestens zwei Befestigungselemente irreversibel vom Gelenkflächenersatzimplantat abtrennbar ausgebildet sind. Beispielsweise kann so ein Operateur das Facettengelenkimplantat noch im Verlauf des chirurgischen Eingriffs individuell für einen Patienten präparieren, indem er zum Beispiel nicht erforderliche oder hinderliche Befestigungselemente vom Gelenkflächenersatzimplantat abtrennt, beispielsweise abreißt oder abschneidet.

Eine weitere, verbesserte individuelle Anpassung des Facettengelenkimplantats an physiologische Gegebenheiten eines Patienten kann insbesondere dadurch erreicht werden, dass mindestens zwei Befestigungselemente in Form unterschiedlicher Arten von Befestigungselementen ausgebildet sind. Beispielsweise kann ein Befestigungselement in Form einer Befestigungslasche, ein anderes in Form eines Befestigungslappens, ein anderes in Form eines Befestigungsstreifens, ein anderes in Form einer Befestigungsschlaufe oder ein anderes in Form einer Befestigungsöse ausgebildet sein. Je nach gewünschter Position des Facettengelenkimplantats am Knochen können unterschiedliche Arten von Befestigungselementen günstiger sein, auch was beispielsweise die Führung des Fixierelements anbelangt.

Um das Abrutschen des Facettengelenkimplantats beim Festlegen mit dem Fixierelement möglichst zu verhindern, kann es günstig sein, wenn das Facettengelenkimplantat mindestens ein Rückhalteelement zum Eingraben in Knochengewebe umfasst. Es können insbesondere zwei, drei oder eine Mehrzahl an Rückhalteelementen vorgesehen sein.

Die Einhaltung der gewünschten Position des Facettengelenkimplantats kann insbesondere weiter dadurch verbessert werden, dass das mindestens eine Rückhalteelement am Gelenkflächenersatzimplantat und/oder an mindestens einem der Befestigungselemente angeordnet oder ausgebildet ist. So kann ein Ab- oder Verrutschen des Facettengelenkimplantats an der Wirbelsäule einfach und sicher verhindert werden. Insbesondere können am Gelenkflächenersatzimplantat und/oder am mindestens einen der Befestigungselemente zwei, drei oder mehr Rückhalteelemente vorgesehen sein.

Besonders einfach werden der Aufbau sowie die Herstellung des Facettengelenkimplantats, wenn das mindestens eine Rückhalteelement in Form eines Widerhakens oder eines spitzen Vorsprungs ausgebildet ist. Beispielsweise kann der Vorsprung nadelförmig oder pyramidenförmig ausgebildet sein.

Damit das Facettengelenkimplantat dauerhaft im Körper verbleiben kann, ist es günstig, wenn es aus einem körperverträglichen Material hergestellt ist. Vorzugsweise sind das Gelenkflächenersatzimplantat und/oder die Befestigungseinrichtung ganz oder teilweise aus einem Metall oder einem Kunststoff hergestellt. Beispielsweise kann es vorteilhaft sein, das Gelenkflächenersatzimplantat aus einem Kunststoff auszubilden und die Befestigungseinrichtung aus einem Metall oder umgekehrt.

Vorteilhafterweise enthält oder ist das Metall Titan oder eine Titanlegierung. Derartige Metalle weisen die zur Ausbildung des Facettengelenkimplantats erforderlichen mechanischen Festigkeiten auf. Zudem kann beispielsweise das Gelenkflächenersatzimplantat in Form eines Metallschaums oder mit einer porösen Oberfläche ausgebildet sein, die das An- und Einwachsen von Knochen verbessern.

Die Herstellung des Facettengelenkimplantats kann insbesondere weiter dadurch vereinfacht werden, dass der Kunststoff Polyetheretherketon und/oder Polyethylen ist oder enthält. Die genannten Kunststoffe eignen sich insbesondere als Implantatwerkstoffe und weisen auch die zur Ausbildung eines Facettengelenkimplantats erforderlichen mechanischen Festigkeiten auf. Bevorzugt ist insbesondere UHMWPE, also Polyethylen mit einem ultrahohen Molekulargewicht.

Günstig kann es ferner sein, wenn der Kunststoff ein thermisch aktivierbarer und/oder thermisch schrumpfbarer Kunststoff ist. Dies gestattet es insbesondere, das Facettengelenkimplantat in gewünschter Weise zu positionieren und daran anschließend durch Wärmeeinleitung ein Schrumpfen, beispielsweise der Befestigungselemente, zu bewirken. Dies kann zu einem Spannen der Befestigungselemente und des Gelenkflächenersatzimplantats relativ zueinander - genutzt werden und so zu einem noch besseren Halt des Facettengelenkimplantats an der Wirbelsäule führen.

Vorteilhaft ist es, wenn aus einem Kunststoff hergestellte Teile oder Bereiche des Gelenkflächenersatzimplantats und/oder der Befestigungseinrichtung mindestens teilweise mit einer osteointegrativen Beschichtung versehen sind. Vorzugsweise ist die osteointegrative Beschichtung am Gelenkflächenersatzimplantat und/oder an der Befestigungseinrichtung an den Stellen oder Bereichen vorgesehen, die bei der Implantation mit Knochen in Kontakt kommen können. Die osteointegrative Beschichtung ermöglicht ein verbessertes An- und Einwachsen von Knochen in das Gelenkflächenersatzimplantat und/oder die Befestigungseinrichtung. Sie kann vorzugsweise porös und aus einem Metall hergestellt sein oder ein Metall oder eine Metalllegierung enthalten. Beispielsweise kann die Beschichtung Titan enthalten. Günstigerweise ist die Beschichtung durch Kaltgasspritzen hergestellt.

Die Handhabbarkeit sowie die Herstellung des Facettengelenkimplantats lassen sich weiter auf einfache Weise dadurch verbessern, dass die mindestens zwei Befestigungselemente in einer Grundstellung eben oder leicht gekrümmt ausgebildet sind.

Um das Facettengelenkimplantat auf einfache Weise durch einen minimalinvasiven Zugang in den Körper eines Patienten einzuführen und in gewünschter Weise individuell an physiologische Gegebenheiten des Patienten anzupassen, ist es vorteilhaft, wenn die mindestens zwei Befestigungselemente flexibel und/oder elastisch ausgebildet sind. Flexibel bedeutet in diesem Sinne insbesondere eine Verformbarkeit dahingehend, dass beispielsweise ein Verbiegen oder Verdrillen der Befestigungselemente möglich ist.

Gemäß einer weiteren bevorzugten Ausführungsform der Erfindung kann ferner vorgesehen sein, dass die mindestens zwei Befestigungselemente und das Gelenkflächenersatzimplantat gelenkig miteinander verbunden sind. So können je nach Ausgestaltung des Facettengelenkimplantats sowie abhängig von der Physiologie des Patienten Zug- und Haltekräfte in optimierter Weise eingeleitet werden.

Vorteilhaft ist es, wenn das Facettengelenkimplantat ein Gelenk zum gelenkigen Verbinden eines der Befestigungselemente mit dem Gelenkflächenersatzimplantat umfasst. Eine solche Ausgestaltung bietet sich insbesondere an, wenn unterschiedliche Materialkombinationen zwischen Gelenkflächenersatzimplantat und Befestigungselement gewünscht werden, um zum Beispiel eine geeignete Gleitpaarung der durch zusammenwirkende Gelenkflächenersatzimplantate definierte Ersatzgelenkflächen bei gleichzeitig flexibler Fixierung auszubilden.

Besonders günstig ist es, wenn das Gelenk in Form eines Scharniergelenks ausgebildet ist. Ein einteiliges Scharniergelenk kann vorzugsweise in Form eines Filmscharniers ausgebildet werden.

Die Herstellung sowie die Stabilität des Facettengelenkimplantats lassen sich insbesondere dadurch verbessern, wenn es insgesamt einstückig ausgebildet ist. Einstückig bedeutet dabei insbesondere, dass das Gelenkflächenersatzimplantat und die mindestens zwei Befestigungselemente einstückig ausgebildet sind. Das Fixierelement kann jedoch als separates Teil vorgesehen sein.

Gemäß einer weiteren bevorzugten Ausführungsform der Erfindung kann vorgesehen sein, dass das Gelenkflächenersatzimplantat und die mindestens zwei Befestigungselemente einstückig ausgebildet sind. So kann beispielsweise das Gelenkflächenersatzimplantat mit den Befestigungselementen in einem einzigen Herstellungsschritt gebildet werden.

Die vorstehende Beschreibung umfasst somit insbesondere die nachfolgend in Form durchnummerierter Sätze definierten Ausführungsformen eines Facettengelenkimplantats:
1. Facettengelenkimplantat (30) umfassend ein Gelenkflächenersatzimplantat (32) und eine Befestigungseinrichtung (38) zum Festlegen des Gelenkflächenersatzimplantats (32) im Gelenkspalt (52) eines Facettengelenks (28) einer menschlichen oder tierischen Wirbelsäule (10), welche Befestigungseinrichtung (38) mindestens zwei vom Gelenkflächenersatzimplantat (32) abstehende Befestigungselemente (40) umfasst, dadurch gekennzeichnet, dass die mindestens zwei Befestigungselemente (40) im Bereich ihrer freien Enden (44) jeweils mit mindestens einer Fixierelementdurchbrechung (48) versehen sind und dass das Facettengelenkimplantat (30) ein Fixierelement (50) umfasst, welches durch mindestens zwei Fixierelementdurchbrechungen (48) von mindestens zwei Befestigungselementen (40) durchgeführt ist und dessen freie Enden (54) miteinander verbindbar sind.
2. Facettengelenkimplantat nach Satz 1, dadurch gekennzeichnet, dass das Fixierelement (50) flexibel ausgebildet ist.
3. Facettengelenkimplantat nach Satz 1 oder 2, dadurch gekennzeichnet, dass das Fixierelement (50) in Form eines Fadens oder eines Drahts ausgebildet ist.
4. Facettengelenkimplantat nach einem der voranstehenden Sätze, dadurch gekennzeichnet, dass die mindestens zwei Befestigungselemente (40) in Form von Befestigungslaschen, -lappen, -streifen, -schlaufen (66) oder - ösen (72) ausgebildet sind.
5. Facettengelenkimplantat nach einem der voranstehenden Sätze, dadurch gekennzeichnet, dass freie Enden (44) der mindestens zwei Befestigungselemente (40) verdickt und/oder verstärkt ausgebildet sind oder dass die mindestens zwei Befestigungselemente (40) im Bereich ihrer freien Enden (44) verdickt und/oder verstärkt ausgebildet sind.
6. Facettengelenkimplantat nach einem der voranstehenden Sätze, dadurch gekennzeichnet, dass die Fixierelementdurchbrechungen (48) verdickt und/oder verstärkt ausgebildet sind.
7. Facettengelenkimplantat nach einem der voranstehenden Sätze, gekennzeichnet durch einen Fixierelementverschluss (56) zum zugbelastbaren Verbinden freier Enden (54) des Fixierelements (50).
8. Facettengelenkimplantat nach Satz 7, dadurch gekennzeichnet, dass der Fixierelementverschluss (56) in Form eines Knotens (58), einer Plombe (60) oder einer Klammer ausgebildet ist.
9. Facettengelenkimplantat nach einem der voranstehenden Sätze, gekennzeichnet durch vier bis 25 Befestigungselemente (40).
10. Facettengelenkimplantat nach einem der voranstehenden Sätze, dadurch gekennzeichnet, dass die mindestens zwei Befestigungselemente (40) unterschiedlich lang und/oder breit und/oder dick ausgebildet sind.
11. Facettengelenkimplantat nach einem der voranstehenden Sätze, dadurch gekennzeichnet, dass die mindestens zwei Befestigungselemente (40) in Form unterschiedlicher Arten von Befestigungselementen (40) ausgebildet sind.
12. Facettengelenkimplantat nach einem der voranstehenden Sätze, gekennzeichnet durch mindestens ein Rückhalteelement (74) zum Eingraben in Knochengewebe.
13. Facettengelenkimplantat nach Satz 12, dadurch gekennzeichnet, dass das mindestens eine Rückhalteelement (74) am Gelenkflächenersatzimplantat (32) und/oder an mindestens einem der Befestigungselemente (40) angeordnet oder ausgebildet ist.
14. Facettengelenkimplantat nach Satz 12 oder 13, dadurch gekennzeichnet, dass das mindestens eine Rückhalteelement (74) in Form eines Widerhakens (78) oder eines spitzen Vorsprungs (76) ausgebildet ist.
15. Facettengelenkimplantat nach einem der voranstehenden Sätze, dadurch gekennzeichnet, dass das Gelenkflächenersatzimplantat (32) und/oder die Befestigungseinrichtung (38) ganz oder teilweise aus einem Metall oder einem Kunststoff hergestellt sind.
16. Facettengelenkimplantat nach Satz 15, dadurch gekennzeichnet, dass der Kunststoff Polyetheretherketon und/oder Polyethylen ist oder enthält.
17. Facettengelenkimplantat nach Satz 15 oder 16, dadurch gekennzeichnet, dass der Kunststoff ein thermisch aktivierbarer und/oder thermisch schrumpfbarer Kunststoff ist.
18. Facettengelenkimplantat nach einem der Sätze 15 bis 17, dadurch gekennzeichnet, dass aus einem Kunststoff hergestellte Teile oder Bereiche des Gelenkflächenersatzimplantats (32) und/oder der Befestigungseinrichtung (38) mit einer osteointegrativen Beschichtung versehen sind.
19. Facettengelenkimplantat nach einem der voranstehenden Sätze, dadurch gekennzeichnet, dass die mindestens zwei Befestigungselemente (40) in einer Grundstellung eben oder leicht gekrümmt ausgebildet sind.
20. Facettengelenkimplantat nach einem der voranstehenden Sätze, dadurch gekennzeichnet, dass die mindestens zwei Befestigungselemente (40) flexibel und/oder elastisch ausgebildet sind.
21. Facettengelenkimplantat nach einem der voranstehenden Sätze, dadurch gekennzeichnet, dass die mindestens zwei Befestigungselemente (40) und das Gelenkflächenersatzimplantat (32) gelenkig miteinander verbunden sind.
22. Facettengelenkimplantat nach Satz 21, gekennzeichnet durch ein Gelenk (80) zum gelenkigen Verbinden eines der Befestigungselemente (40) mit dem Gelenkflächenersatzimplantat (32).
23. Facettengelenkimplantat nach Satz 22, dadurch gekennzeichnet, dass das Gelenk (80) in Form eines Scharniergelenks (84), insbesondere in Form eines Filmscharniers (82) ausgebildet ist.
24. Facettengelenkimplantat nach einem der voranstehenden Sätze, dadurch gekennzeichnet, dass es einstückig ausgebildet ist.
25. Facettengelenkimplantat nach einem der Sätze 1 bis 24, dadurch gekennzeichnet, dass das Gelenkflächenersatzimplantat (32) und die mindestens zwei Befestigungselemente (40) einstückig ausgebildet sind.

Die nachfolgende Beschreibung bevorzugter Ausführungsformen der Erfindung dient im Zusammenhang mit den Zeichnungen der näheren Erläuterung. Es zeigen:
- Figur 1:: eine schematische Ansicht eines Bewegungssegments einer menschlichen Wirbelsäule mit einem Facettengelenkimplantat;
- Figur 2:: eine vergrößerte Ansicht des Bereichs A in Figur 1;
- Figur 3:: eine perspektivische Ansicht des in den Figuren 1 und 2 dargestellten Facettengelenkimplantats in einer Grundstellung;
- Figur 4:: eine schematische Ansicht eines weiteren Ausführungsbeispiels eines Befestigungselements für ein Facettengelenkimplantats;
- Figur 5:: eine schematische Ansicht eines weiteren Ausführungsbeispiels eines Befestigungselements für ein Facettengelenkimplantat;
- Figur 6:: eine schematische Ansicht eines weiteren Ausführungsbeispiels eines Fixierelementverschlusses für ein Facettengelenkimplantat;
- Figur 7:: eine ausschnittsweise schematische Schnittansicht durch ein Gelenkflächenersatzimplantat eines Facettengelenkimplantats;
- Figur 8:: eine schematische Ansicht eines weiteren Ausführungsbeispiels eines Befestigungselements für ein Facettengelenkimplantat;
- Figur 9:: eine schematische Ansicht eines weiteren Ausführungsbeispiels eines Befestigungselements für ein Facettengelenkimplantat;
- Figur 10:: eine schematische Ansicht eines weiteren Ausführungsbeispiels eines Gelenkflächenersatzimplantats für ein Facettengelenkimplantat;
- Figur 11:: eine schematische Ansicht eines zwischen einem Befestigungselement und einem Gelenkflächenersatzimplantat eines Facettengelenkimplantats ausgebildetes Filmscharnier; und

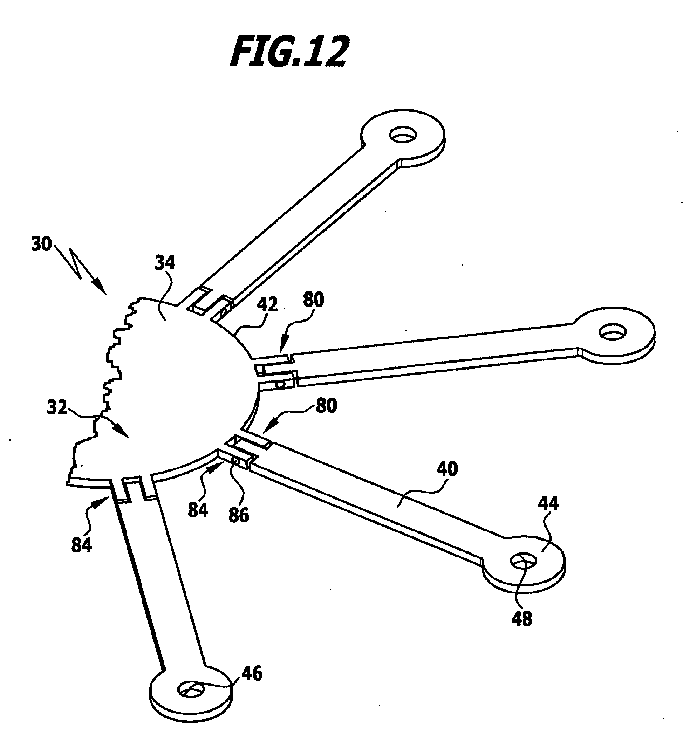

- Figur 12:: eine schematische Ansicht eines zwischen einem Befestigungselement und einem Gelenkflächenersatzimplantat eines Facettengelenkimplantats ausgebildetes Scharniergelenks.

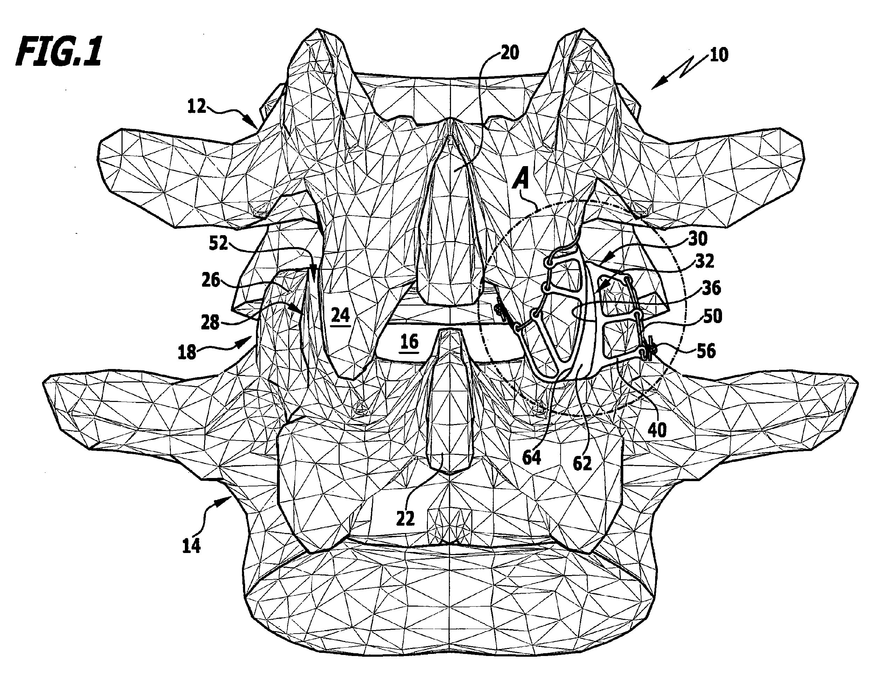

In Figur 1 ist schematisch ein Teil einer menschlichen Wirbelsäule 10 dargestellt. Die beiden benachbarten Wirbel 12 und 14 zusammen mit der zwischen ihnen angeordneten Bandscheibe 16 bilden ein insgesamt mit dem Bezugszeichen 18 bezeichnetes Bewegungssegment der Wirbelsäule 10 aus. Seitlich neben den Dornfortsätzen 20 und 22 der Wirbel 12 und 14 stehen in dorsaler Richtung und aufeinander zu weisend Fortsätze 24 und 26 ab, welche zusammen jeweils ein Facettengelenk 28 bilden. Symmetrisch zur Medianebene umfasst somit jedes Bewegungssegment 18 zwei Facettengelenke 28.

Das in Figur 1 rechts dargestellte Facettengelenk 28 wurde zur Behandlung einer Facettengelenkarthrose oder -degeneration durch ein beziehungsweise zwei Facettengelenkimplantate 30 teilweise ersetzt. Jedes Facettengelenkimplantat 30 umfasst ein Gelenkflächenersatzimplantat 32, welches eine an einem der Fortsätze 24 beziehungsweise 26 anliegende Anlagefläche 34 sowie eine in entgegengesetzter Richtung, also in Richtung auf den jeweils anderen Fortsatz 24, 26 hin, weisende Gelenkfläche 36 umfasst. Wird an jedem Fortsatz 24 und 26 ein Facettengelenkimplantat 30 angebracht, wie in Figur 1 schematisch rechts im Bereich A dargestellt, so weisen jeweils die Gelenkflächen 36 der Gelenkflächenersatzimplantate 32 aufeinander zu.

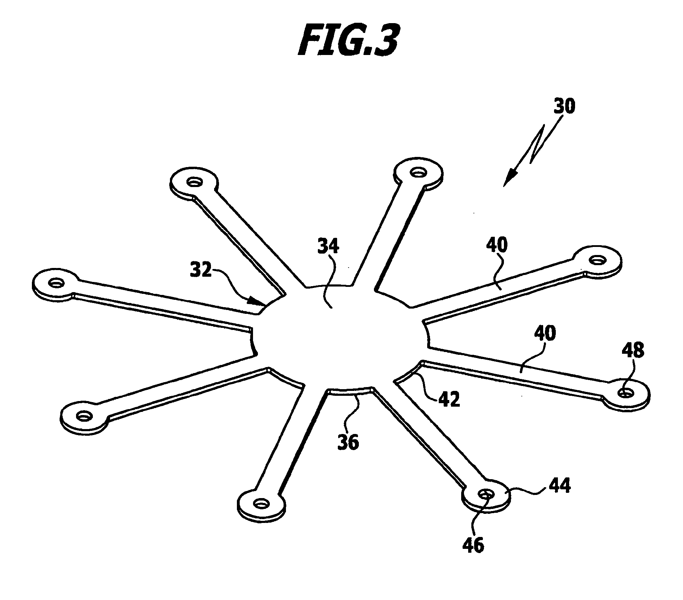

Zur Befestigung des Facettengelenkimplantats 30 am Fortsatz 24, 26 dient eine Befestigungseinrichtung 38. Diese umfasst mindestens zwei, vorzugsweise vier bis 25 Befestigungselemente 40, die von einem Rand 42 des im Wesentlichen in Form einer Kreisscheibe ausgebildeten Gelenkflächenersatzimplantats 32 abstehend ausgebildet sind. Das Facettengelenkimplantat 30 ist insgesamt einstückig ausgebildet und in einer in Figur 3 dargestellten Grundstellung, insbesondere direkt nach seiner Herstellung, im Wesentlichen eben.

Die Befestigungselemente 40 sind an ihrem freien Ende 44 etwas im Außendurchmesser verdickt und weisen eine Durchbrechung 46 in Form einer Bohrung auf. Diese bildet eine Fixierelementdurchbrechung 48.

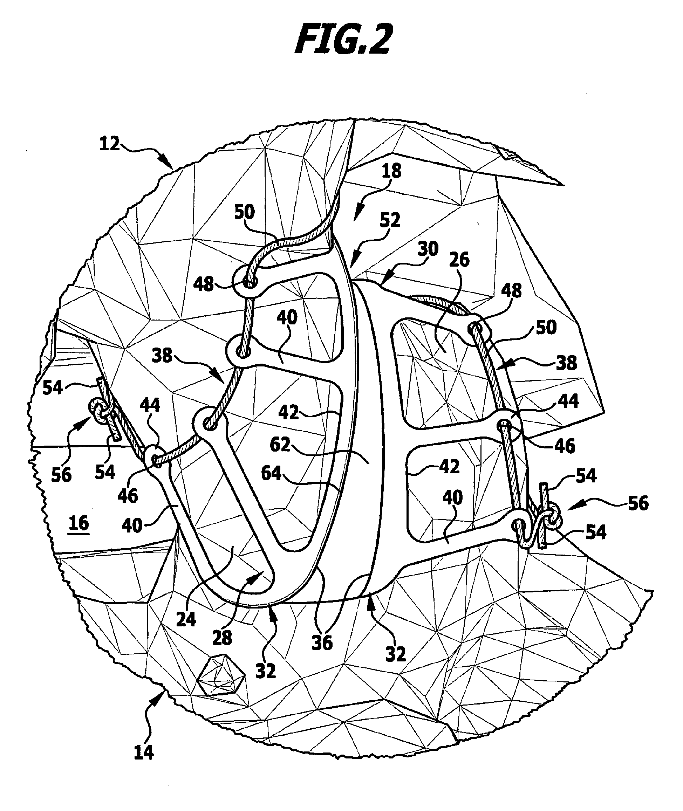

Wie schematisch in Figur 2 dargestellt und dort gut zu erkennen, ist ein Fixierelement 50 in Form eines flexiblen Fadens oder Drahts vorgesehen, welches durch alle Fixierelementdurchbrechungen 48 geführt ist.

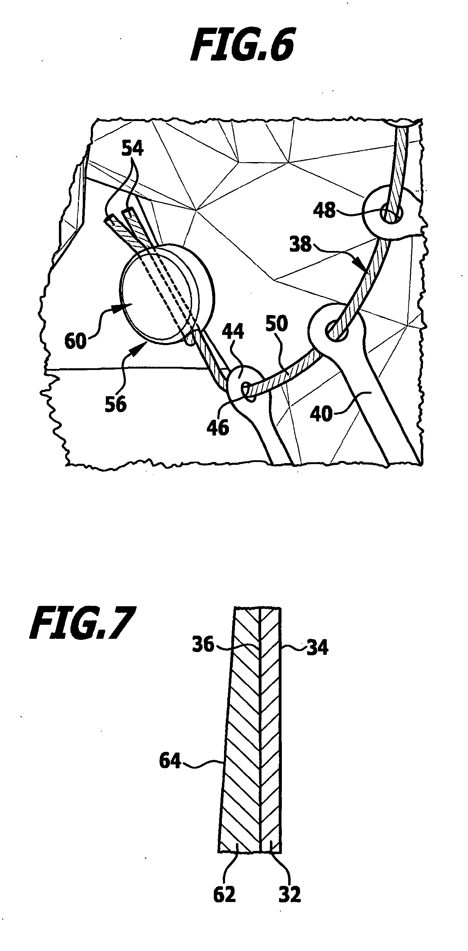

Zum Befestigen des Facettengelenkimplantats 30 wird jeweils die Anlagefläche 34 des Gelenkflächenersatzimplantats 32 an die geschädigte oder degenerierte Gelenkfläche eines der Fortsätze 24 beziehungsweise 26 in den Gelenkspalt 52 des Facettengelenks 28 herangeführt. Die geschädigte Gelenkfläche kann vorher zusätzlich präpariert werden. Die Befestigungselemente 40 werden vom jeweils anderen Fortsatz 24, 26 weg gebogen und das durch die Fixierelementdurchbrechungen 48 geführte Fixierelement 50 um den jeweiligen Fortsatz 24, 26 gespannt. Freie Enden 54 des Fixierelements 50 werden miteinander verbunden. Dies kann mittels eines zugbelastbaren Fixierelementverschlusses 56 geschehen, beispielsweise in Form eines Knotens, wie schematisch in den Figuren 1 und 2 dargestellt, oder in Form einer insbesondere aufschiebbaren oder aufcrimpbaren Plombe 60, wie schematisch in Figur 6 dargestellt. Alternativ sind auch Klammern zum Verbinden der freien Enden 54 denkbar.

Das Gelenkflächenersatzimplantat 32 kann wie in Figur 3 schematisch dargestellt in Form einer flachen Scheibe ausgebildet sein. Alternativ kann die Scheibe auch auf der Gelenkfläche 36 ein Abstandselement 62 tragen, welches wiederum eine eigene Gelenkfläche 64 definiert, welche vom Gelenkflächenersatzimplantat 32 und dessen Gelenkfläche 36 weg weist. Dies ist schematisch in Figur 7 als Teilschnittansicht dargestellt. So kann beispielsweise die Gelenkfläche 64 des Abstandselements 62 an der Gelenkfläche 36 eines korrespondierenden Facettengelenkimplantats 30 anliegen. Das Abstandselement kann zylindrisch und/oder keilförmig ausgebildet sein.

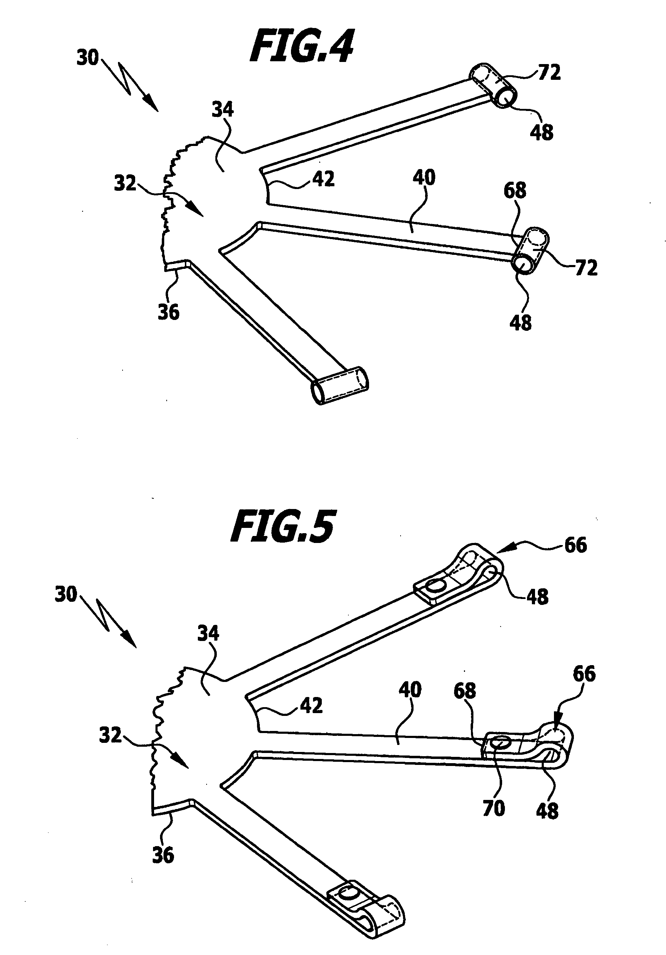

Vom Gelenkflächenersatzimplantat weg weisende Enden der Befestigungselemente 40 können auch, wie in Figur 5 schematisch dargestellt, in Form einer Schlaufe 66 ausgebildet sein. Ein freies Ende 68 des Befestigungselements 40 ist dabei auf sich selbst zurückgeführt und beispielsweise mittels eines Niets 70 oder einer Punktschweißung befestigt. Durch die Schlaufe 66 wird wiederum eine Fixierelementdurchbrechung 48 definiert, die in einer Richtung parallel zu einer vom Gelenkflächenersatzimplantat 32 definierten Ebene orientiert ist.

Eine weitere Variante einer Fixierelementdurchbrechung 48 ist schematisch in Figur 4 dargestellt. An ein freies Ende 68 des streifen, oder laschenförmigen Befestigungselements 40 ist eine hülsenförmige Öse 72 angeformt oder angebracht, welche eine ebenfalls in einer Richtung parallel zu der vom Gelenkflächenersatzimplantat 32 definierten Ebene orientierte Fixierelementdurchbrechung 48 definiert.

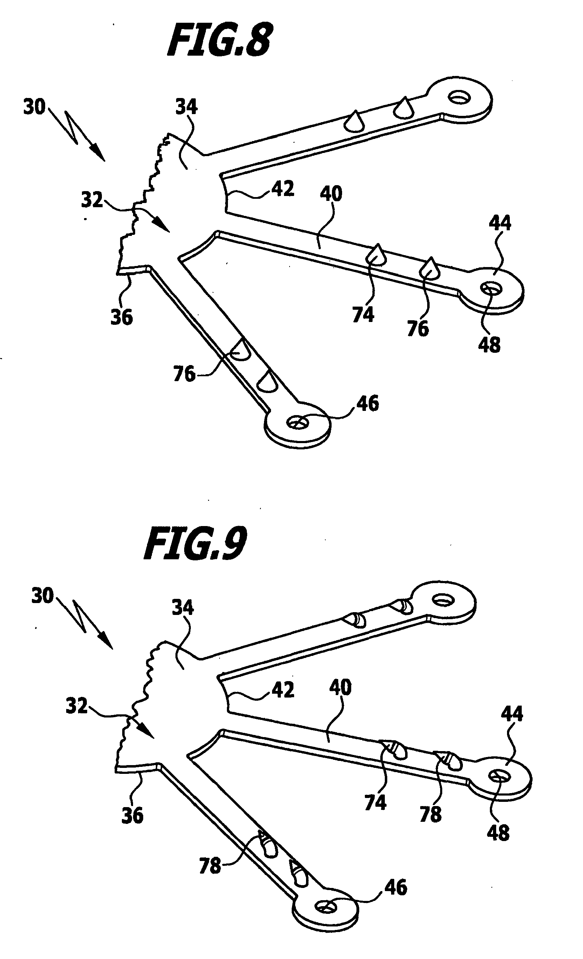

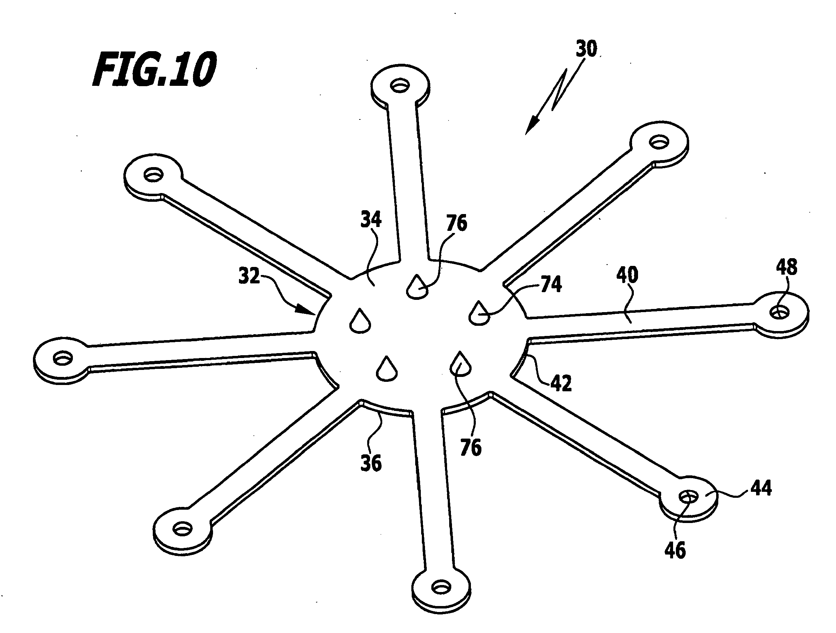

Um ein Verrutschen des Facettengelenkimplantats 30 an den Fortsätzen 24 beziehungsweise 26 möglichst ganz ausschließen zu können, ist es günstig, wenn das Facettengelenkimplantat 30 ein oder mehrere Rückhalteelemente 74 trägt. Diese können, wie schematisch in Figur 10 dargestellt, beispielsweise auf der Anlagefläche 34 angeordnet und von dieser senkrecht oder im Wesentlichen senkrecht abstehend ausgebildet sein. Des Weiteren können die Rückhalteelemente 74 auch, wie schematisch in den Figuren 8 und 9 dargestellt, in Form von spitzen Vorsprüngen 76 oder Widerhaken 78 an einem Befestigungselement 40 abstehend angeordnet oder ausgebildet sein. Die Vorsprünge 76 können insbesondere in Form von Kegelspitzen oder Pyramidenspitzen ausgebildet sein. Es können zwei, drei, vier, fünf oder mehr Rückhalteelemente 74 von der Anlagefläche 34 abstehen. Jedes Befestigungselement 40 kann ein, zwei, drei, vier oder mehr Rückhalteelemente 74 aufweisen.

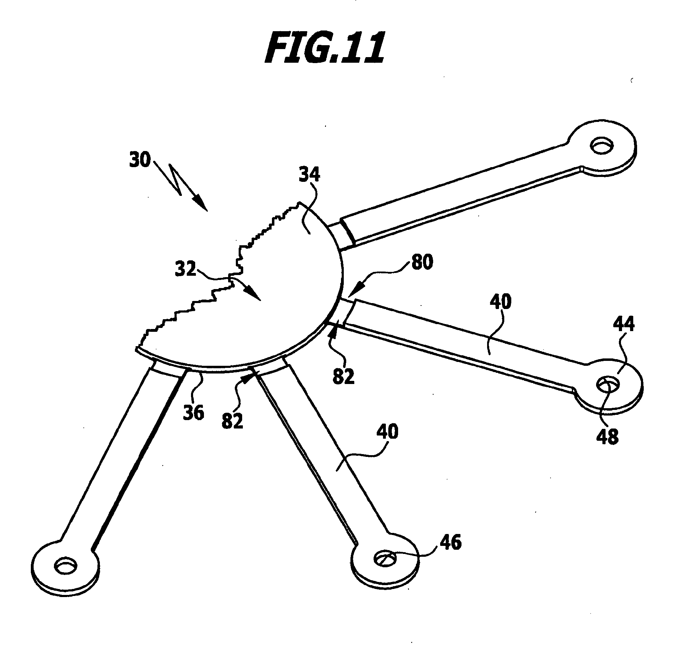

Zur Verbesserung einer Beweglichkeit und somit einer Verformbarkeit des Facettengelenkimplantats 30 zur bestmöglichen Anpassung an die Fortsätze 24, 26 kann zwischen dem Gelenkflächenersatzimplantat 32 und dem Befestigungselement 40 ein Gelenk 80 ausgebildet sein. Dabei kann es sich um ein wie in Figur 11 schematisch dargestelltes Filmscharnier 82 handeln, welches durch eine Schwächung im Bereich zwischen dem Befestigungselement 40 und dem Gelenkflächenersatzimplantat 32 ausgebildet wird. Alternativ kann das Gelenk 80 auch in Form eines echten Scharniergelenks 84 ausgebildet sein. Dieses kann insbesondere einen Gelenkstift 86 umfassen, welcher eine nicht näher dargestellte Aussparung am Gelenkflächenersatzimplantat 32 durchsetzt und welcher eine Lagerwelle für eine am Befestigungselement 40 vorgesehene, zum Gelenkstift 86 korrespondierende Lagerdurchbrechung bildet. So ist eine Verschwenkung des Befestigungselements 40 um eine vom Gelenkstift 86 definierte Schwenkachse möglich.

Die oben beschriebenen unterschiedlichen Ausgestaltungen der Befestigungselemente 40, insbesondere auch deren unterschiedliche Fixierelementdurchbrechungen 48, können an einem Facettengelenkimplantat 30 beliebig miteinander kombiniert werden. Die Befestigungselemente können zudem unterschiedlich dick und/oder lang ausgeführt sein. Denkbar ist es ferner, das Facettengelenkimplantat 30 so auszubilden, dass einzelne Befestigungselemente 40 beim Implantieren entfernt, beispielsweise abgeschnitten oder abgerissen werden können. Ferner können die freien Enden 44 und 68 der Befestigungselemente 40 optional auch verstärkt oder verdickt ausgebildet werden, um ein Ausreißen in Folge einer Zugbelastung mittels des Fixierelements 50 zu vermeiden.

Nicht dargestellt sind ferner weitere Optionen, um eine Befestigung der beschriebenen Facettengelenkimplantate 30 an den Fortsätzen 24 und 26 zu verbessern. Um das Einbringen des Gelenkflächenersatzimplantats 32 in den Gelenkspalt 52 zu ermöglichen, muss die Kapsel des Facettengelenks 28 zumindest teilweise entfernt werden. Die Präparation des Facettengelenks 28 richtet sich insbesondere nach der vorliegenden Knochenstruktur des Patienten, das heißt in einigen Fällen kann es erforderlich sein, den Knochen durch einen oder mehrere kleine Kerben zu präparieren. Diese Kerben wirken unterstützend zur Verhinderung eines späteren Abrutschens der Befestigungseinrichtung 38, insbesondere des Fixierelements 50. Sie verwachsen im Anschluss an den Eingriff, da der abgetragene Knochen vom Körper nachgebildet wird. Alternativ sind zur Erhöhung der Stabilität des eingesetzten Facettengelenkimplantats 30 auch weitere Befestigungselemente, wie beispielsweise Knochenpins oder -schrauben denkbar, über welche das Fixierelement 50 zusätzlich geführt werden kann oder die die Befestigungselemente 40 zusätzlich am Knochen fixieren.

Selbstverständlich können pro Facettengelenkimplantat 30 auch zwei oder mehr Fixierelemente 50 vorgesehen sein, die jeweils nur durch einen Teil der Fixierelementdurchbrechungen 48 geführt und mit sich selbst oder einem weiteren Fixierelement 50 verbunden werden können.

Die Befestigungseinrichtung 38 beziehungsweise das Facettengelenkimplantat 30 ist in allen gängigen Implantatmaterialien herstellbar. Zum Beispiel kommen hierfür Titan, Titanlegierungen, Polyetheretherketon, Polyethylen mit ultrahohem Molekulargewicht (UHMWPE) in Frage. Des Weiteren können diese Implantatmaterialien auch zusätzlich mit geeigneten Beschichtungen versehen sein, um das Anwachsen von Knochen zu ermöglichen und ein Verrutschen des Gelenkflächenersatzimplantats 32 an den Fortsätzen 24, 26 möglichst zu verhindern. Darüber hinaus wäre es auch vorstellbar, ein Implantatmaterial, insbesondere einen Kunststoff, zu verwenden, welches nach dem Einbringen durch Wärme zum Schrumpfen gebracht wird. Nachdem das Facettengelenkimplantat 30 positioniert wäre, würde man durch Wärmeeinleitung ein Schrumpfen beispielsweise der Befestigungselemente 40 bewirken können, was so zu einem Spannen der Befestigungselemente 40 und des Gelenkflächenersatzimplantats 32 relativ zueinander führen und so für einen noch besseren Halt sorgen würde.

Das Einbringen des Facettengelenkimplantats 30 kann also insbesondere als eine Form des Überziehens eines Fortsatzes 24, 26 mit einer Hülle oder Haube über einen zylindrischen Gegenstand verglichen werden. Die Befestigungselemente 40 sind zur Einbringung vorzugsweise eben oder leicht gekrümmt ausgebildet.

Alle beschriebenen Facettengelenkimplantate 30 lassen sich einfach und sicher handhaben. Durch den einfachen Zugmechanismus der Befestigungseinrichtung 38 lässt sich eine Primärstabilität des künstlichen Facettengelenks gewährleisten, ohne umliegende Strukturen zu beschädigen. Die Befestigung oder Fixierung des Facettengelenkimplantats 30 kann mit wenigen Schritten an knöchernen Strukturen des Patienten, also insbesondere an die Fortsätze 24, 26 angepasst werden. Allenfalls in manchen Fällen kann eine Präparation am Knochen aufgrund der morphologischen Vielfalt der Knochenstruktur erforderlich sein, beschränkt sich jedoch auf die bereits erwähnten kleinen Kerben am Knochen.

Die vorgeschlagenen Facettengelenkimplantate 30 weisen eine äußerst flexible Gestalt auf und ermöglichen die Anpassung an nahezu jede erdenkliche Gelenkfläche. Das Einbringen der Facettengelenkimplantate 30 in den Körper kann durch einen für den Patienten schonenden, minimalinvasiven Eingriff erfolgen. Die oben beschriebene Implantation ermöglicht einen maximalen Erhalt von knöcherner Struktur, so dass insbesondere für Revisionseingriffe wichtige Wirbelkörperstrukturen, wie zum Beispiel die Pedikel der Wirbel, nicht geschädigt werden müssen. Alle beschriebenen Konstruktionen sind kostengünstig in der Herstellung, einfach und funktionssicher. Sie erlauben eine einfache Handhabung und eine Verriegelung, das heißt Befestigung, insbesondere auch von dorsal.

Unabhängig von einer Form oder konkreten Ausgestaltung können insbesondere alle oben beschriebenen Facettengelenkimplantate 30, insbesondere wenn sie ganz oder teilweise aus einem Kunststoff hergestellt ist, vollständig oder teilweise mit einer osteointegrativen Beschichtung 88 versehen sein. Vorzugsweise ist die osteointegrative Beschichtung 88 am Gelenkflächenersatzimplantat 32 und/oder an der Befestigungseinrichtung 38 auf äußeren Flächen an den Stellen oder Bereichen vorgesehen, die bei der Implantation mit Knochen in Kontakt kommen können. Dies ist schematisch in Figur 3 dargestellt. Die osteointegrative Beschichtung88 ermöglicht ein verbessertes An- und Einwachsen von Knochen in das Gelenkflächenersatzimplantat und/oder die Befestigungseinrichtung. Sie kann vorzugsweise porös und aus einem Metall hergestellt sein oder ein Metall oder eine Metalllegierung enthalten. Beispielsweise kann die Beschichtung Titan enthalten. Günstigerweise ist die Beschichtung 88 durch Kaltgasspritzen hergestellt.

## Patentansprüche

1. Facettengelenkimplantat (30) umfassend ein Gelenkflächenersatzimplantat (32) und eine Befestigungseinrichtung (38) zum Festlegen des Gelenkflächenersatzimplantats (32) im Gelenkspalt (52) eines Facettengelenks (28) einer menschlichen oder tierischen Wirbelsäule (10), welche Befestigungseinrichtung (38) mindestens zwei vom Gelenkflächenersatzimplantat (32) abstehende Befestigungselemente (40) umfasst, **dadurch gekennzeichnet, dass** die mindestens zwei Befestigungselemente (40) im Bereich ihrer freien Enden (44) jeweils mit mindestens einer Fixierelementdurchbrechung (48) versehen sind und dass das Facettengelenkimplantat (30) ein Fixierelement (50) umfasst, welches durch mindestens zwei Fixierelementdurchbrechungen (48) von mindestens zwei Befestigungselementen (40) durchgeführt ist und dessen freie Enden (54) miteinander verbindbar sind.

2. Facettengelenkimplantat nach Anspruch 1, **dadurch gekennzeichnet, dass** freie Enden (44) der mindestens zwei Befestigungselemente (40) verdickt und/oder verstärkt ausgebildet sind oder dass die mindestens zwei Befestigungselemente (40) im Bereich ihrer freien Enden (44) verdickt und/oder verstärkt ausgebildet sind.

3. Facettengelenkimplantat nach einem der voranstehenden Ansprüche,
**dadurch gekennzeichnet, dass** die Fixierelementdurchbrechungen (48) verdickt und/oder verstärkt ausgebildet sind.

4. Facettengelenkimplantat nach einem der voranstehenden Ansprüche,
**dadurch gekennzeichnet, dass** die mindestens zwei Befestigungselemente (40) unterschiedlich lang und/oder breit und/oder dick ausgebildet sind.

5. Facettengelenkimplantat nach einem der voranstehenden Ansprüche,
**dadurch gekennzeichnet, dass** die mindestens zwei Befestigungselemente (40) in Form unterschiedlicher Arten von Befestigungselementen (40) ausgebildet sind.

6. Facettengelenkimplantat nach einem der voranstehenden Ansprüche,
**gekennzeichnet durch** mindestens ein Rückhalteelement (74) zum Eingraben in Knochengewebe.

7. Facettengelenkimplantat nach Anspruch 6, **dadurch gekennzeichnet, dass** das mindestens eine Rückhalteelement (74) am Gelenkflächenersatzimplantat (32) und/oder an mindestens einem der Befestigungselemente (40) angeordnet oder ausgebildet ist.

8. Facettengelenkimplantat nach einem der voranstehenden Ansprüche,
**dadurch gekennzeichnet, dass** das Gelenkflächenersatzimplantat (32) und/oder die Befestigungseinrichtung (38) ganz oder teilweise aus einem Metall oder einem Kunststoff hergestellt sind.

9. Facettengelenkimplantat nach Anspruch 8, **dadurch gekennzeichnet, dass** der Kunststoff Polyetheretherketon und/oder Polyethylen ist oder enthält.

10. Facettengelenkimplantat nach Anspruch 8 oder 9, **dadurch gekennzeichnet, dass** der Kunststoff ein thermisch aktivierbarer und/oder thermisch schrumpfbarer Kunststoff ist.

11. Facettengelenkimplantat nach einem der Ansprüche 8 bis 10, **dadurch gekennzeichnet, dass** aus einem Kunststoff hergestellte Teile oder Bereiche des Gelenkflächenersatzimplantats (32) und/oder der Befestigungseinrichtung (38) mit einer osteointegrativen Beschichtung versehen sind.

12. Facettengelenkimplantat nach einem der voranstehenden Ansprüche,
**dadurch gekennzeichnet, dass** die mindestens zwei Befestigungselemente (40) in einer Grundstellung eben oder leicht gekrümmt ausgebildet sind.

13. Facettengelenkimplantat nach einem der voranstehenden Ansprüche, **dadurch gekennzeichnet, dass** die mindestens zwei Befestigungselemente (40) und das Gelenkflächenersatzimplantat (32) gelenkig miteinander verbunden sind.

14. Facettengelenkimplantat nach Anspruch 13, **gekennzeichnet durch** ein Gelenk (80) zum gelenkigen Verbinden eines der Befestigungselemente (40) mit dem Gelenkflächenersatzimplantat (32).

15. Facettengelenkimplantat nach Anspruch 14, **dadurch gekennzeichnet, dass** das Gelenk (80) in Form eines Scharniergelenks (84), insbesondere in Form eines Filmscharniers (82) ausgebildet ist.

## Claims

1. A facet joint implant (30) comprising a joint surface replacement implant (32) and a fastening device (38) for fixing the joint surface replacement implant (32) in the joint gap (52) of a facet joint (28) of a human or animal spinal column (10), which fastening device (38) comprises at least two fastening elements (40) that protrude from the joint surface replacement implant (32), **characterized in that** the at least two fastening elements (40) are each provided in the region of their free ends (44) with at least one fixing element through hole (48), and **in that** the facet joint implant (30) comprises a fixing element (50) which is fed through at least two fixing element through holes (48) of at least two fastening elements (40), wherein the free ends of said fixing element are connectable to one another (54).

2. A facet joint implant in accordance with Claim 1, **characterized in that** free ends (44) of the at least two fastening elements (40) are thickened and/or reinforced or **in that** the at least two fastening elements (40) are thickened and/or reinforced in the region of their free ends (44).

3. A facet joint implant in accordance with any of the preceding Claims, **characterized in that** the fixing element through holes (48) are thickened and/or reinforced.

4. A facet joint implant in accordance with any of the preceding Claims, **characterized in that** the at least two fastening elements (40) are of differing length and/or breadth and/or thickness.

5. A facet joint implant in accordance with any of the preceding Claims, **characterized in that** the at least two fastening elements (40) are in the form of different kinds of fastening elements (40).

6. A facet joint implant in accordance with any of the preceding Claims, **characterized by** at least one retaining element (74) for digging into bone tissue.

7. A facet joint implant in accordance with Claim 6, **characterized in that** the at least one retaining element (74) is arranged or formed on the joint surface replacement implant (32) and/or on at least one of the fastening elements (40).

8. A facet joint implant in accordance with any of the preceding Claims, **characterized in that** the joint surface replacement implant (32) and/or the fastening device (38) are made entirely or partly of a metal or a synthetic material.

9. A facet joint implant in accordance with Claim 8, **characterized in that** the synthetic material is or contains polyetheretherketone and/or polyethylene.

10. A facet joint implant in accordance with Claim 8 or 9, **characterized in that** the synthetic material is a thermally activatable and/or a thermally shrinkable synthetic material.

11. A facet joint implant in accordance with any of the Claims 8 to 10, **characterized in that** parts or regions of the joint surface replacement implant (32) and/or the fastening device (38) that are made from a synthetic material are provided with an osteo-integrative coating.

12. A facet joint implant in accordance with any of the preceding Claims, **characterized in that** the at least two fastening elements (40) are flat or slightly curved in a basic position.

13. A facet joint implant in accordance with any of the preceding Claims, **characterized in that** the at least two fastening elements (40) and the joint surface replacement implant (32) are connected to one another in articulated manner.

14. A facet joint implant in accordance with Claim 13, **characterized by** a joint (80) for the articulated connection of one of the fastening elements (40) to the joint surface replacement implant (32).

15. A facet joint implant in accordance with Claim 14, **characterized in that** the joint (80) is in the form of a hinge joint (84), and in particular, in the form of a film hinge (82).

## Revendications

1. Implant d'articulation facettaire (30), comprenant un implant de substitution de surface articulaire (32) et un dispositif d'attache (38) pour immobiliser 1"implant de substitution de surface articulaire (32) dans l'interstice articulaire (52) d'une articulation facettaire (28) d'une colonne vertébrale (10) humaine ou animale, ledit dispositif d'attache (38) comprenant au moins deux éléments d'attache (40) s'écartant de l'implant de substitution de surface articulaire (32), **caractérisé en ce que** lesdits au moins deux éléments d'attache (40) sont pourvus, dans la zone de leurs extrémités libres (44), respectivement d'au moins un passage d'élément de fixation (48), et **en ce que** l'implant d'articulation facettaire (30) comprend un élément de fixation (50), que l'on fait passer à travers au moins deux passages d'élément de fixation (48) d'au moins deux éléments d'attache (40), et dont les extrémités libres (54) peuvent être reliées mutuellement.

2. Implant d'articulation facettaire selon la revendication 1, **caractérisé en ce que** des extrémités libres (44) desdits au moins deux éléments d'attache (40) sont d'une configuration plus épaisse et/ou renforcée, ou **en ce que** lesdits au moins deux éléments d'attache (40) sont réalisés de manière plus épaisse et/ou renforcée dans la zone de leurs extrémités libres (44).

3. Implant d'articulation facettaire selon l'une des revendications précédentes, **caractérisé en ce que** les passages d'élément de fixation (48) sont d'une configuration plus épaisse et/ou renforcée.

4. Implant d'articulation facettaire selon l'une des revendications précédentes, **caractérisé en ce que** lesdits au moins deux éléments d'attache (40) sont réalisés d'une longueur différente et/ou d'une largeur différente et/ou d'une épaisseur différente.

5. Implant d'articulation facettaire selon l'une des revendications précédentes, **caractérisé en ce que** lesdits au moins deux éléments d'attache (40) sont réalisés sous la forme d'éléments d'attache (40) de type différent.

6. Implant d'articulation facettaire selon l'une des revendications précédentes, **caractérisé par** au moins un élément de retenue (74) destiné à s'incruster dans le tissu osseux.

7. Implant d'articulation facettaire selon la revendication 6, **caractérisé en ce que** ledit au moins un élément de retenue (74) est agencé et/ou réalisé sur 1"implant de substitution de surface articulaire (32) et/ou sur au moins l'un des éléments d'attache (40).

8. Implant d'articulation facettaire selon l'une des revendications précédentes, **caractérisé en ce que** 1"implant de substitution de surface articulaire (32) et/ou le dispositif d'attache (38) sont fabriqués en totalité ou en partie en un métal ou une matière plastique.

9. Implant d'articulation facettaire selon la revendication 8, **caractérisé en ce que** la matière plastique est constituée par, ou renferme du polyétheréther-cétone et/ou du polyéthylène.

10. Implant d'articulation facettaire selon la revendication 8 ou la revendication 9, **caractérisé en ce que** la matière plastique est une matière plastique pouvant être thermo-activée et/ou thermo-rétractable.

11. Implant d'articulation facettaire selon 1"une des revendications 8 à 10, **caractérisé en ce que** des parties ou des zones fabriquées en matière plastique, de 1"implant de substitution de surface articulaire (32) et/ou du dispositif d'attache (38), sont pourvues d'un revêtement d'ostéointégration.

12. Implant d'articulation facettaire selon l'une des revendications précédentes, **caractérisé en ce que** lesdits au moins deux éléments d'attache (40) sont, dans une position initiale, d'une configuration plane ou légèrement courbée.

13. Implant d'articulation facettaire selon l'une des revendications précédentes, **caractérisé en ce que** lesdits au moins deux éléments d'attache (40) et l'implant de substitution de surface articulaire (32) sont reliés mutuellement de manière articulée.

14. Implant d'articulation facettaire selon la revendication 13, **caractérisé par** une articulation (80) pour la liaison articulée de l'un des éléments d'attache (40) avec l'implant de substitution de surface articulaire (32).

15. Implant d'articulation facettaire selon la revendication 14, **caractérisé en ce que** l'articulation (80) est réalisée sous la forme d'une articulation à charnière (84), notamment sous la forme d'une charnière à film (82).
